(19) Europäisches Patentamt
European Patent Office
Office européen des brevets

(11) EP 4 592 273 A1

(12) **EUROPÄISCHE PATENTANMELDUNG**

(43) Veröffentlichungstag:
30.07.2025 Patentblatt 2025/31

(21) Anmeldenummer: 24196456.8

(22) Anmeldetag: 26.08.2024

(51) Internationale Patentklassifikation (IPC):
C07C 67/303 (2006.01)    C07C 69/80 (2006.01)
C07C 67/03 (2006.01)    C07C 67/08 (2006.01)

(52) Gemeinsame Patentklassifikation (CPC):
(C-Sets verfügbar)
C07C 67/303; C07C 67/03; C07C 67/08;
C07C 2601/14    (Forts.)

(84) Benannte Vertragsstaaten:
AL AT BE BG CH CY CZ DE DK EE ES FI FR GB
GR HR HU IE IS IT LI LT LU LV MC ME MK MT NL
NO PL PT RO RS SE SI SK SM TR
Benannte Erstreckungsstaaten:
BA
Benannte Validierungsstaaten:
GE KH MA MD TN

(30) Priorität: 29.01.2024 EP 24154409

(71) Anmelder: Evonik Oxeno GmbH & Co. KG
45772 Marl (DE)

(72) Erfinder:
• van Eickels, Michael
45657 Recklinghausen (DE)

• Grass, Michael
45721 Haltern am See (DE)
• Geilen, Frank
45721 Haltern am See (DE)
• Simon, Berno
45772 Marl (DE)
• Zanthoff, Horst-Werner
45481 Mülheim a.d. Ruhr (DE)
• Becker, Tobias
45721 Haltern am See (DE)

(74) Vertreter: Evonik Patent Association
c/o Evonik Industries AG
IP Management
Postcode 84/339
Rodenbacher Chaussee 4
63457 Hanau (DE)

(54) **VERFAHREN ZUR HERSTELLUNG VON 1,2-CYCLOHEXANDICARBONSÄUREDIALKYLESTERN**

(57) Gegenstand der Erfindung ist ein Verfahren zur Herstellung von 1,2-Cyclohexandicarbonsäuredialkylestern durch Kernhydrieren des entsprechenden Dialkylphthalats, welches eine CO-Zahl von weniger als 0,1 mg KOH/g aufweist. Die Erfindung hat außerdem die Verwendung der so hergestellten 1,2-Cyclohexandicarbonsäuredialkylester als Weichmacher oder als Teil einer Weichmacherzusammensetzung für Kunststoffe, insbesondere PVC zum Gegenstand.

EP 4 592 273 A1

(52) Gemeinsame Patentklassifikation (CPC): (Forts.)

C-Sets
**C07C 67/03, C07C 69/80;**
**C07C 67/08, C07C 69/80;**
**C07C 67/303, C07C 69/75**

**Beschreibung**

Die vorliegende Erfindung betrifft ein Verfahren zur Herstellung von 1,2-Cyclohexandicarbonsäuredialkylestern durch Kernhydrieren des entsprechenden Dialkylphthalats, welches eine CO-Zahl von weniger als 0,1 mg KOH/g aufweist. Die Erfindung hat außerdem die Verwendung der so hergestellten 1,2-Cyclohexandicarbonsäuredialkylester als Weichmacher oder als Teil einer Weichmacherzusammensetzung für Kunststoffe, insbesondere PVC zum Gegenstand.

Weichmacher werden in vielen technischen Bereich eingesetzt, um Kunststoffe wie Polyvinylchlorid (PVC) weicher und biegsamer zu machen. Seit vielen Jahren sind Phthalate, also die Diester der (ortho-)Phthalsäure, die dominierende Weichmacherklasse. In den letzten Jahren haben aber auch die Alkylester von Cyclohexandicarbonsäuren an Bedeutung gewonnen, nicht zuletzt wegen der Diskussion um eventuelle Gesundheitsbedenken der Phthalat-basierten Weichmacher. Dabei spielen vor allem die 1,2-Cyclohexandicarbonsäuredialkylester und neuerdings auch die 1,4-Cyclohexandicarbonsäuredialkylester eine Rolle.

1,2-, 1,3- und 1,4-Cyclohexandialkyldicarbonsäureester können durch die Hydrierung des aromatischen Rings der entsprechenden Phthalate, Isophthalate oder Terephthalate (nachfolgend: Kernhydrierung) hergestellt werden. Teilweise werden solche Kernhydrierungen bereits großtechnisch durchgeführt, beispielsweise für die Herstellung von DINCH, dem Diisononylester der 1,2-Cyclohexandicarbonsäure.

Die Reaktionsgeschwindigkeit der Kernhydrierung spielt dabei eine wichtige Rolle für die Wirtschaftlichkeit des Verfahrens, da sowohl die Investitions- als auch die Betriebskosten hiervon beeinflusst werden.

Die Aufgabe der vorliegenden Erfindung bestand deshalb darin, ein Verfahren zur Herstellung von 1,2-Cyclohexandialkyldicarbonsäureestern bereitzustellen, mit dem die Hydriergeschwindigkeit auf höherem Niveau gehalten und somit die Produktivität des Hydrierprozesses gesteigert werden kann. Weiterhin sollte die Katalysatordesaktivierung reduziert werden.

Überraschend wurde nun gefunden, dass Dialkylphthalate, bei denen die Alkylgruppen jeweils 8 oder 9 Kohlenstoffatome aufweisen, sich leichter und schneller hydrieren lassen, wenn deren Carbonylzahl (CO-Zahl) einen bestimmten Wert nicht überschreitet. Werden also entsprechende Dialkylphthalate bei der Kernhydrierung eingesetzt, steigt die Produktivität und damit auch die Wirtschaftlichkeit des Verfahrens. Weiterhin wird die Menge an den Hydrierkatalysator desaktivierenden Komponenten reduziert, wodurch die Katalysatoraktivität für einen längeren Zeitraum auf einem höheren Niveau gehalten werden kann.

Das erfindungsgemäße Verfahren ist demgemäß ein Verfahren zur Herstellung von 1,2-Cyclohexandicarbonsäuredialkylestern, bei denen die beiden Alkylgruppen jeweils 8 oder 9 Kohlenstoffatome aufweisen, wobei das Verfahren mindestens die folgenden Schritte umfasst

a) Herstellen eines Dialkylphthalats durch Umsetzung von Phthalsäureanhydrid oder Dimethylphthalat mit einem C8- oder C9-Alkohol und Ausschleusen eines Teils einer während der Reaktion durch Anlegen eines Unterdrucks im Bereich von 500 bis 900 mbar absolut abdestillierten Leichtsiederphase, die zumindest nicht umgesetzten Alkohol und Reaktionsnebenprodukte umfasst,

b) Kernhydrieren des in Schritt a) hergestellten Dialkylphthalats, bei dem die beiden Alkylgruppen jeweils 8 oder 9 Kohlenstoffatome aufweisen, in Anwesenheit eines heterogenen Hydrierkatalysators mit einem Wasserstoff-haltigen Gas zum entsprechenden 1,2-Cyclohexandicarbonsäuredialkylester umfasst, dadurch gekennzeichnet, dass

das bei der Kernhydrierung in Schritt b) eingesetzte Dialkylphthalat eine CO-Zahl von weniger als 0,1 mg KOH/g, vorzugsweise weniger als 0,08 mg KOH/g, weiterhin bevorzugt weniger als 0,07 mg KOH/g und besonders bevorzugt weniger als 0,01 mg KOH/g aufweist.

Die CO-Zahl ist definiert als die Menge KOH in Milligramm, die der zur Oximierung von 1 g Substanz notwendigen Menge Hydroxylamin äquivalent sind. Die CO-Zahl wird darüber bestimmt, dass die in carbonylfreiem Alkohol gelöste Substanz mit einem Überschuss an Hydroxylamin zum entsprechenden Oxim umgesetzt und das unverbrauchte Hydroxylamin mit Salzsäure zurücktitriert wird.

Zur Bestimmung der CO-Zahl muss zunächst der Äquivalenzpunkt mit einer Kalibrierlösung bestimmt werden. Dazu werden Kalibrierlösungen mit unterschiedlichen Mengen an Cyclohexanon in einem geeigneten Lösemittel, z. B. carbonylfreiem Methanol hergestellt. Die theoretische CO-Zahl ergibt sich aus der nachfolgenden Formel:

$$\text{CO-Zahl (theoretisch)} = \frac{\text{Molmasse } KOH \cdot \text{Reinheit } Cyclohexanon}{\text{Molmasse } Cyclohexanon}$$

Die hergestellten Kalibrierlösungen werden jeweils mit 0,1 mol/l Salzsäure titriert. Der gemessene pH-Wert wird dann als Funktion des jeweiligen Salzsäureverbrauchs aufgetragen und der Äquivalenzpunkt bestimmt. Damit ist das System kalibriert.

Die Bestimmung der CO-Zahl einer unbekannten Probe kann dann wie folgt bestimmt werden. Dazu wird zunächst eine geeignete Probenmenge in ein Reaktionsgefäß vorgelegt und in 50 ml eines geeigneten Lösemittels, z. B. carbonylfreiem Methanol gelöst. Mit dem eingesetzten Lösemittel, z. B. Methanol muss vorher eine Blindwertbestimmung ohne Probe wie nachfolgend beschrieben durchgeführt werden. Die Lösung der Probe in dem Lösemittel, z. B. Methanol wird mit Bromphenolblau versetzt und der pH-Wert - sofern erforderlich - durch Zugabe von Salzsäure oder Natronlauge so angepasst, dass eine grün-gelbe Färbung der Lösung (entspricht einem pH-Wert von etwa 3) vorhanden ist. Anschließend werden 20 ml Hydroxylaminlösung (c = 0,24 mol/l) zudosiert und die entstandene Lösung im Reaktionsgefäß 1 h unter Rückfluss gekocht.

Nach dem Abkühlen auf Raumtemperatur wird der Rückflusskühler mit 10 ml des Lösemittels, z. B. carbonylfreiem Methanol gespült und anschließend die Reaktionslösung mit 0,1 mol/l Salzsäure bis zum Äquivalenzpunkt titriert.

Die CO-Zahl kann dann anhand der nachfolgenden Formel berechnet werden:

$$\text{CO-Zahl (mg KOH/g)} = \frac{(V_B - V_H) \cdot F_{HCl} \cdot c_{HCl} \cdot M_{KOH}}{E_P}$$

wobei $V_B$ der Salzsäureverbrauch bei der Blindwertbestimmung in ml, $V_H$ der Salzsäureverbrauch bei der zu untersuchenden Probe in ml, $F_{HCl}$ der Titer von Salzsäure, $c_{HCl}$ die Konzentration der Salzsäure in mol/l, $M_{KOH}$ die Molmassen von KOH = 56,11 g/mol und $E_P$ die Probeneinwaage in g ist.

Sofern darauf geachtet wird, dass das bei der Kernhydrierung in Schritt b) eingesetzte Dialkylphthalat eine CO-Zahl von weniger als 0,1 aufweist, lässt sich die Kernhydrierung schneller durchführen. Von den dadurch hergestellten 1,2-Cyclohexandicarbonsäuredialkylestern, bei denen die Alkylgruppen jeweils 8 oder 9 Kohlenstoffatome aufweisen, sind die 1,2-Cyclohexandicarbonsäuredialkylester bevorzugt, bei denen die Alkylgruppen 2-Ethylhexyl oder Isononyl sind. Weiterhin bevorzugt sind Dialkylphthalate, bei denen die beiden Alkylgruppen die gleiche Anzahl an Kohlenstoffatomen aufweisen. Bevorzugt werden deswegen Di-2-ethylhexylphthalat oder Di-isononylphthalat eingesetzt, woraus bei der Kernhydrierung 1,2-Cyclohexandicarbonsäure-di-2-ethylhexylester oder 1,2-Cyclohexandicarbonsäure-diisononylester (DINCH) entstehen. Besonders bevorzugt entsteht als Produkt bei der erfindungsgemäßen Kernhydrierung 1,2-Cyclohexandicarbonsäure-diisononylester (DINCH).

Um entsprechende Dialkylphthalate für die Kernhydrierung in Schritt b) zu erhalten, wird das Herstellverfahren gemäß Schritt a) der vorliegenden Erfindung eingesetzt. Die Herstellung des Dialkylphthalats erfolgt dabei durch Umsetzung von Phthalsäureanhydrid oder Dimethylphthalat mit einem C8- oder C9-Alkohol und Ausschleusen eines Teils einer während der Reaktion durch Anlegen eines Unterdrucks im Bereich von 500 bis 900 mbar absolut abdestillierten Leichtsiederphase, die zumindest nicht umgesetzten Alkohol und Reaktionsnebenprodukte umfasst. Das Ausschleusen der Reaktionsnebenprodukte kann für die Reduktion der CO-Zahl sorgen.

Die Herstellung der erfindungsgemäßen Dialkylphthalate durch Veresterung von Phthalsäureanhydrid mit einem Alkohol oder einem Alkoholgemisch mit 8 oder 9 Kohlenstoffatomen kann nach allen bekannten Verfahren durchgeführt werden. Vorzugsweise erfolgt die Veresterung allerdings nach einem Verfahren, bei dem das Reaktionswasser durch azeotrope Destillation mit dem Alkohol entfernt wird und die durch die azeotrope Destillation aus der Reaktion entfernte Flüssigkeitsmenge vollständig oder teilweise mit dem Einsatzalkohol wieder ergänzt wird. Als Flüssigkeitsmenge wird im Folgenden das durch azeotrope Destillation aus der Reaktion entfernte Flüssigkeitsvolumen, hauptsächlich bestehend aus Reaktionswasser und Alkohol, bezeichnet. Ein vollständiger Ersatz der entfernten Flüssigkeitsmenge ist bevorzugt.

Die Veresterung mit Phthalsäureanhydrid zu den Dialkylphthalaten kann erfindungsgemäß autokatalysiert oder säure- bzw. basenkatalysiert durchgeführt werden. Als Veresterungskatalysatoren können Lewis- oder Brönstedsäuren oder metallorganische Stoffe, eingesetzt werden. Bevorzugte Veresterungskatalysatoren sind Alkoholate, Sulfonsäuren, Carbonsäuresalze oder Chelatverbindungen von Titan oder Zirkonium, wobei das Katalysatormolekül ein oder mehrere Metallatome enthalten kann. Insbesondere werden Tetra(isopropyl)orthotitanat und Tetra(butyl)orthotitanat eingesetzt. Die Katalysatorkonzentration hängt von der Art des Katalysators ab. Bei den bevorzugt eingesetzten Titanverbindungen beträgt diese 0,005 bis 1,0 Gew.-% bezogen auf das Reaktionsgemisch, insbesondere 0,01 bis 0,3 Gew.-%.

Die erfindungsgemäße Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Der Katalysator kann in reiner Form oder als Lösung, bevorzugt gelöst in einem der Einsatzstoffe, zu Beginn oder erst nach Erreichen der Reaktionstemperatur eingebracht werden. Der umzusetzende Alkohol, der als Schleppmittel dient, kann im stöchiometrischen Überschuss eingesetzt werden. Bevorzugt wird ein Überschuss von 5 bis 50 %, besonders bevorzugt 10 bis 30 % bezogen auf das eingesetzte Phthalsäureanhydrid eingesetzt.

Die Reaktionstemperaturen bei der Veresterung liegen bei Verwendung von Titankatalysatoren zwischen 120 °C und 270 °C, vorzugsweise zwischen 130 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperatur erhöhen die Reaktionsgeschwindigkeiten und begünstigen Nebenreaktionen, wie beispielsweise Wasserabspaltung aus Alkoholen oder Bildung farbiger Nebenprodukte.

Die Umesterung von Dimethylphthalat mit einem C8- oder C9-Alkohol ist ebenfalls ein bekanntes Verfahren und bereits im Stand der Technik beschrieben, beispielsweise in der WO 2009/095126 A1. Die Umesterung findet üblicherweise in flüssiger Phase statt. Dabei wird das Dimethylphthalat mit dem jeweiligen Alkohol oder den jeweiligen Alkoholgemischen in Gegenwart eines geeigneten Katalysators umgesetzt. Das bei der Veresterung entstehende Methanol wird vorzugsweise schon während der Reaktion abgetrennt. Da die Reaktionstemperaturen üblicherweise über dem Siedepunkt von Methanol liegen, kann das Methanol einfach dampfförmig abgetrennt werden.

Der zur Herstellung des Dialkylphthalats bei der Veresterung oder der Umesterung eingesetzte Alkohol ist ein Alkohol oder ein Alkoholgemisch mit 8 oder 9 Kohlenstoffatomen. Bevorzugt ist der bei der Veresterung eingesetzte Alkohol 2-Ethylhexanol oder Isononanol. Besonders bevorzugt ist der Alkohol Isononanol. Isononanol wird im Rahmen der vorliegenden Erfindung als ein Gemisch von primären C9-Alkoholen verstanden, die sowohl linear oder verzweigt sein können.

Die erfindungsgemäße Veresterung oder erfindungsgemäße Umesterung in Schritt a) wird vorzugsweise diskontinuierlich durchgeführt. Bei der hier bevorzugten diskontinuierlichen Betriebsweise handelt es sich insbesondere um eine Batchproduktion. Es wird also eine durch das Reaktorvolumen begrenzte Menge hergestellt und dann die Reaktion beendet. Nach Entleerung des Reaktors kann anschließend eine neue Batchproduktion gestartet werden.

Als Reaktoren eigenen sich dem Fachmann bekannte Reaktoren, die für die Synthese von Dialkylphthalaten eingesetzt werden können. Geeignete Reaktoren umfassen insbesondere eine Destillationskolonne, über die das gebildete Wasser oder das gebildete Methanol und nach der Reaktion auch die nicht umgesetzten Alkohole abgetrennt werden. Die Destillationskolonne ist vorzugsweise fest mit dem Reaktor oder den Reaktoren des erfindungsgemäßen Verfahrens verbunden. Der Reaktor stellt dabei quasi den Sumpf der Destillationskolonne dar. Durch die vorherrschenden Reaktionstemperaturen können Wasser oder Methanol schon während der Veresterung oder der Umesterung in die Gasphase übergehen und über die Destillationskolonne abdestilliert werden. Die dadurch verlorene Flüssigkeitsmenge kann durch Zuführen des eingesetzten Alkohols oder der eingesetzten Alkoholmischung ersetzt werden.

Die Temperatur bei der Herstellung der Dialkylphthalate ist von verschiedenen Faktoren abhängig, beispielweise von der durchgeführten Reaktionsvariante, beträgt aber vorzugsweise 150 °C bis 260 °C. Dieser Temperaturbereich ist sowohl für die Veresterung als auch für die Umesterung geeignet. Der Druck bei Veresterung und bei der Umesterung liegt vorzugsweise im Bereich von 0,5 bis 10 bar absolut. Temperatur und Druck sind vorzugsweise so einzustellen, dass das entstehende Wasser oder das entstehende Methanol schon während der Reaktion abdestilliert werden kann.

Am Ende der Reaktion liegen die hergestellten Dialkylphthalate in einer Reaktionslösung vor, die neben den hergestellten Verbindungen auch zumindest die nicht umgesetzten Alkohole oder die nicht umgesetzte Alkoholmischung umfasst. Da bei der Veresterung oder der Umesterung Nebenprodukte entstehen können, die leichter sieden als die eingesetzten Alkohole oder Alkoholmischungen, sind in der Regel auch solche Reaktionsnebenprodukte in der Reaktionslösung vorhanden. Mögliche Nebenprodukte sind durch abgespaltene Wassergruppen entstandene Alkene oder durch Abspaltung zumindest einer Säuregruppe bzw. Estergruppe entstandene Monocarbonsäure(ester), die zu einer Erhöhung der CO-Zahl führen können.

Wie erwähnt wird während der Reaktion bereits eine Leichtsiederphase abdestilliert, die vornehmlich Wasser und Alkohol, z. B. der nicht umgesetzte Alkohol bei der Veresterung oder der nicht umgesetzte Alkohol und/oder Methanol bei der Umesterung. Zum Ende der Reaktion hin werden aufgrund des steigenden Umsatzes die Mengen an durch die Ver- bzw. Umesterung entstehendem Wasser oder Methanol geringer. Die Abtrennung kann durch Anlegen eines Unterdrucks von 500 bis 800 mbar absolut, vorzugsweise 520 bis 750 mbar absolut unterstützt werden. Das hat auch den Vorteil dass entstandene Reaktionsnebenprodukte mit der Leichtsiederphase übergehen. Deshalb wird zumindest ein Teil der Leichtsiederphase ausgeschleust und dadurch die Menge an Reaktionsnebenprodukten in der (finalen) Reaktionslösung verringert.

Der Unterdruck wird vorzugsweise erst bei Erreichen eines Schwellenwerts des Reaktionsumsatzes angelegt. In einer bevorzugten Ausführungsform wird der Unterdruck nach Erreichen eines Reaktionsumsatzes von mindestens 92%, besonders bevorzugt von mindestens 95%, wird der erwähnte Unterdruck angelegt. Dazu wird der Reaktionsumsatz schon während der Reaktion überwacht. Es ist aber auch möglich, dass nicht der Umsatz, sondern ein einziger anderer oder mehrere andere Parameter während der Reaktion überwacht werden, wobei der oder die Parameter, ggf. nach vorheriger Kalibrierung, einen Rückschluss auf den Umsatz oder allgemeiner den Fortschritt der Reaktion zulassen.

Das Anlegen des Unterdrucks kann durch bekannte Apparate oder Maschinen erreicht werden. Ein Beispiel für eine solche geeignete Apparatur ist eine (Vakuum-)Pumpe, mit der der Unterdruck im Reaktor erzeugt wird. Die Temperatur bei Anlegen des Unterdrucks kann der Reaktionstemperatur entsprechen. Erfolgt das Anlegen des Unterdrucks erst mit einer gewissen Verzögerung nach Erreichen des Schwellenwerts des Umsatzes kann die Temperatur auch geringer sein als die Reaktionstemperatur.

Der bei der Abdestillation der Leichtsiederphase zuerst übergehende Teil der Leichtsiederphase, also der Teil der Leichtsiederphase, der nach Anlegen des Unterdrucks als erstes abdestilliert wird, wird zumindest teilweise ausgeschleust und verworfen. Bezogen auf die Menge der gesamten abdestillierten Leichtsiederphase, die zur Erzeugung des Rohprodukts abgetrennt wird, beträgt die Menge des ausgeschleusten Teils des Leichtsiederphase vorzugsweise 0,05 bis 8 Gew.-%, vorzugsweise 0,1 bis 4 Gew.-%.

Die Kernhydrierung von Dialkylphthalaten gemäß Schritt b) ist dem Fachmann grundsätzlich bekannt. Die Kernhydrierung wird mit einem Wasserstoff-haltigen Gas durchgeführt. Als Wasserstoff-haltiges Gas können grundsätzlich beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Es können auch Gasgemische mit Inertgasen eingesetzt werden. Als Wasserstoff-haltiges Gas wird bevorzugt Wasserstoff in einer Reinheit $\geq 95$ %, insbesondere $\geq 98$ % eingesetzt. Inertgasanteile können beispielsweise Stickstoff oder Methan sein. Das Wasserstoff-haltige Gas wird vorzugsweise so eingesetzt, dass der Wasserstoff im Überschuss, insbesondere in einem Überschuss von bis zu 200 %, bevorzugt in einem Überschuss von 5 bis 100 % und besonders bevorzugt in einem Überschuss von 10 bis 50 % bezogen auf die stöchiometrische Menge, die zur Erzielung des gewünschten Umsatzes benötigt wird, vorliegt.

Bei der erfindungsgemäßen Kernhydrierung in Schritt b) werden zudem heterogene Hydrierkatalysatoren eingesetzt, die vorzugsweise mindestens ein Übergangsmetall, besonders bevorzugt ein Metall der Gruppe 8 des Periodensystems der Elemente enthalten. Vorzugsweise werden als Übergangsmetall Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird. Neben den bereits genannten Metallen kann zusätzlich mindestens ein Metall der Gruppe 7 und 11 des Periodensystems der Elemente in den Katalysatoren enthalten sein. Bevorzugt werden Rhenium und/oder Kupfer eingesetzt.

Der Gehalt an Übergangsmetall im erfindungsgemäßen Hydrierkatalysator liegt vorzugsweise im Bereich von 0,1 bis 10 Gew.-%, insbesondere im Bereich von 0,3 bis 5 Gew.-%, ganz besonders im Bereich von 0,5 bis 3 Gew.-%.

Bevorzugt sind die eingesetzten heterogen Hydrierkatalysatoren Trägerkatalysatoren, d. h. sie umfassen ein Trägermaterial. Als Trägermaterial können Aktivkohle, Siliciumcarbid, Aluminium-oxid, Siliciumdioxid, Alumosilikat, Titandioxid, Zirkoniumdioxid, Magnesiumoxid, Zinkoxid oder Mischungen davon verwendet werden. Besonders bevorzugt wird Titandioxid oder Aluminiumoxid als Trägermaterial eingesetzt. Zusätzlich können diese Trägermaterialien Alkalimetalle, Erdalkalimetalle und/oder Schwefel enthalten.

Die erfindungsgemäße Kernhydrierung der Dialkylphthalate wird vorzugsweise in mindestens einer Hydriereinheit durchgeführt. Unter einer Hydriereinheit wird in der vorliegenden Erfindung eine Einheit verstanden, die einen Reaktor oder mehrere Reaktoren, die parallel und/oder in Reihe geschaltet vorliegen können, umfassen, also einen Reaktor oder eine Reaktoranordnung, in der die Kernhydrierung stattfindet. In einer besonders bevorzugten Ausführungsform wird die Kernhydrierung in mindestens zwei hintereinandergeschalteten Hydriereinheiten durchgeführt, wobei mindestens eine der beiden Hydriereinheiten in Schlaufenfahrweise, d. h. unter Rückführung eines Teils des jeweiligen Hydrieraustrags, betrieben wird. Es kann vorteilhaft sein, wenn alle der mindestens zwei Hydriereinheiten bei der Kernhydrierung in Schlaufenfahrweise betrieben werden. Ebenso vorteilhaft kann es sein, wenn die letzte Hydriereinheit im geraden Durchgang betrieben wird. In eine besonders bevorzugten Ausführungsform der vorliegenden Erfindung wird die Kernhydrierung in zwei hintereinandergeschalteten Hydriereinheiten durchgeführt, wobei die erste Hydriereinheit in Schlaufenfahrweise und die letzte Hydriereinheit im geraden Durchgang betrieben wird.

In einer alternativen Ausführungsform wird die Kernhydrierung in mindestens drei hintereinandergeschalteten Hydriereinheiten durchgeführt, wobei mindestens die ersten beiden Hydriereinheiten in Schlaufenfahrweise betrieben werden. Auch die letzte Hydriereinheit kann in Schlaufenfahrweise betrieben werden, was also einer Ausführungsform entspricht, bei der alle der mindestens drei Hydriereinheiten in Schlaufenfahrweise betrieben werden. Ebenso kann die letzte Hydriereinheit im geraden Durchgang betrieben werden.

Eine weitere besonders bevorzugte Ausführungsform ist die parallele Anordnung der Reaktoren, beispielsweise in einem Rohrbündelreaktor.

Die einzelnen Reaktoren können dabei adiabatisch, polytrop oder praktisch isotherm, d. h. mit einem Temperaturanstieg (Differenz der Temperatur am Einlass und der Temperatur am Auslass des Reaktors) von typischerweise kleiner als 15 K betrieben werden. Dabei werden insbesondere die in Schlaufenfahrweise betriebenen Reaktoren bevorzugt quasi isotherm gefahren, also vorzugsweise mit einem Temperaturanstieg kleiner 15 K betrieben. Bei Reaktoren, die nicht in Schlaufenfahrweise betrieben werden, liegt der bevorzugte Temperaturanstieg im Reaktor bei unter 35 K, besonders bevorzugt unter 25 K. Zwischen einzelnen Hydrierelementen kann eine Kühlvorrichtung angebracht sein, um die Temperatur vor dem Eintritt in die folgende Hydriereinheit abzusenken.

Die erfindungsgemäße Kernhydrierung der Dialkylphthalate wird bevorzugt in der Flüssig/Gas-Mischphase oder Flüssigphase in Dreiphasenreaktoren im Gleichstrom durchgeführt, wobei das Wasserstoff-haltige Gas in an sich bekannter Weise im flüssigen Edukt/Produktstrom verteilt wird. Im Interesse einer gleichmäßigen Flüssigkeitsverteilung, einer verbesserten Reaktionswärmeabfuhr und/oder einer hohen Raum-Zeit-Ausbeute werden die in Schlaufenfahrweise betriebenen Reaktoren vorzugsweise mit hohen Flüssigkeitsbelastungen von 10 bis 400, bevorzugt von 20 bis 200 und besonders bevorzugt von 40 bis 150 m³ pro m² Querschnitt des leeren Reaktors und Stunde gefahren. Die Flüssigkeitsbelastungen können in den in Schlaufenfahrweise betriebenen Reaktoren gleich oder verschieden sein. Vorzugsweise ist die Flüssigkeitsbelastung im ersten Reaktor am größten und nimmt in den nachfolgenden in Schlaufenfahrweise betriebenen Reaktoren ab. Einer oder mehrere Reaktoren können dabei teilweise mit Flüssigkeit geflutet sein oder komplett als Rieselbettreaktoren fungieren.

Die Kernhydrierung der Dialkylphthalate kann in Abwesenheit oder in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten. Folgende Substanzen können als Lösemittel bei der Kernhydrierung eingesetzt werden: Geradkettige oder cyclische Ether, wie beispielsweise Tetrahydrofuran oder Dioxan, sowie aliphatische Alkohole, in denen der Alkylrest 1 bis 13 Kohlenstoffatome aufweist. Bevorzugt als Lösemittel verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole. Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Eduktkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben.

Bevorzugt liegt der Eduktgehalt im Reaktorzulauf zwischen 1 und 70 %, Der gewünschte Konzentrationsbereich kann bei denjenigen Reaktoren, die in Schlaufenfahrweise betrieben werden, durch das Kreislaufverhältnis (Mengenverhältnis von rückgeführten Hydrieraustrag zu Edukt) eingestellt werden. Die Eduktkonzentrationen im Reaktorzulauf nehmen vorzugsweise vom ersten bis zum letzten Reaktor ab.

Eine andere Möglichkeit zur Temperaturkontrolle ist die Verdünnung des zur Hydrierung verwendeten Wasserstoff-haltigen Gases, insbesondere des Wasserstoffs mit einem Inertgas. Das Inertgas kann dabei aus der Gruppe, bestehend aus Stickstoff, Helium, Neon, Argon, Kohlendioxid und Mischungen daraus, ausgewählt werden. Bevorzugt werden Stickstoff oder Kohlendioxid eingesetzt, da diese am einfachsten und günstigsten verfügbar sind. Besonders bevorzugt ist Stickstoff das Inertgas für das erfindungsgemäße Verfahren. Durch die Verdünnung des zur Hydrierung verwendeten Wasserstoff-haltigen Gases kann die Reaktion verlangsamt und dadurch die Temperatur kontrolliert werden. Dieses Verfahren eignet sich auch zum Anfahren des Reaktors nach Einbau oder Regeneration des Katalysators wenn kein geeignetes Lösemittel vorhanden ist.

Die Kernhydrierung der Dialkylphthalate wird erfindungsgemäß bevorzugt in einem Druckbereich von 3 bis 300 bar, insbesondere von 15 bis 200 bar, ganz besonders bevorzugt von 50 bis 200 bar durchgeführt. Der Druck kann in den einzelnen Reaktoren gleich oder verschieden sein. Vorzugsweise sind die Drücke gleich oder annähernd gleich, d. h. weichen maximal um 10% voneinander ab.

Die Hydriertemperaturen liegen bei der Kernhydrierung bevorzugt im Bereich von 50 bis 250 °C, vorzugsweise im Bereich von 80 bis 200 °C. Die Hydriertemperaturen können in einzelnen Reaktoren gleich oder verschieden sein.

Als Produkte des erfindungsgemäßen Verfahrens werden entsprechende Zusammensetzungen erhalten, die von den Einsatzstoffen und dem Umsatz bei der Hydrierung abhängig sind. Die Zusammensetzung, die bei der erfindungsgemäßen Kernhydrierung entsteht, hat vorzugsweise einen Gehalt an 1,2-Cyclohexandicarbonsäuredialkylestern von über 96 Gew.-%, insbesondere von über 98 Gew.-%, besonders bevorzugt von über 99 Gew.-%. Dieses Gemisch kann direkt oder nach Reinigung eingesetzt werden. Die Abtrennung von Nebenprodukten kann beispielsweise durch Destillation oder durch Strippen mit Wasserdampf oder mit einem Inertgas wie Stickstoff erfolgen. Bevorzugt werden geringe Mengen an Leichtsieder durch Strippen mit Wasserdampf im Temperaturbereich von 120 °C bis 240 °C, insbesondere im Bereich von 150 bis 200 °C und vorzugsweise bei einem Druck von 0,05 bis 0,1 bar abgetrennt.

Die erfindungsgemäß hergestellten 1,2-Cyclohexandicarbonsäuredialkylester, bei denen die beiden Alkylgruppen jeweils 8 oder 9 Kohlenstoffatome aufweisen, können vorteilhaft als Weichmacher oder Teil einer Weichmacherzusammensetzung in Kunststoffen oder Kunststoffzusammensetzungen, als Zusatz in Farben oder Lacken, in Klebstoffen oder Klebstoffkomponenten, in Dichtungsmassen oder als Lösemittel verwendet werden.

Die hergestellten 1,2-Cyclohexandicarbonsäuredialkylester können auch in Mischungen mit anderen Weichmachern, insbesondere sogenannten Schnellgelierern, als Weichmacher eingesetzt werden. Der Anteil an erfindungsgemäßen 1,2-Cyclohexandialkylestern in dem Gemisch mit anderen Weichmachern beträgt vorzugsweise 15 bis 95 Gew.-%, besonders bevorzugt 20 bis 90 Gew.-% und ganz besonders bevorzugt 25 bis 85 Gew.-%, wobei sich die Anteile aller vorhandenen Weichmacher zu 100 Gew.-% addieren. Die genannten Zusammensetzungen aus 1,2-Cyclohexandialkylestern und anderen Weichmachern können als Weichmacherzusammensetzung in Kunststoffen und Kunststoffzusammensetzungen, Klebstoffen, Dichtungsmassen, Lacken, Farben, Plastisolen oder Tinten verwendet werden.

Die Kunststoffzusammensetzungen, die die hergestellten 1,2-Cyclohexandicarbonsäuredialkylester enthalten können, können Polymere, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten enthalten. Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Besonders bevorzugt ist die Verwendung von PVC.

Bevorzugt enthält die erfindungsgemäße Kunststoffzusammensetzung als PVC-Typ Suspensions-, Masse-, Mikrosuspensions- oder Emulsions-PVC. Bezogen auf 100 Gewichtsteile Polymer enthalten die erfindungsgemäßen Zusammensetzungen vorzugsweise von 5 bis 200, bevorzugt von 10 bis 150 Gewichtsteile an erfindungsgemäßem Weichmacher.

Die Kunststoffzusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl, Gleitmittel, Treibmittel, Kicker, Antioxidanzien, Rheologie-Additive oder Biozide.

Die erfindungsgemäßen Kunststoffzusammensetzungen aus den 1,2-Cyclohexandicarbonsäuredialkylestern und den vorgenannten Polymermaterialien können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Dachmembranen, Tapeten oder Tinten oder zu deren Herstellung verwendet werden.

Mit den Weichmacherzusammensetzungen hergestellte Kunststoffprodukte können beispielsweise Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Dachmembranen, Tapeten, Kabel und Drahtummantelungen sein. Bevorzugte Anwendungsgebiete aus dieser Gruppe sind Lebensmittelverpackungen, Spielzeug, Medizinalartikel, Tapeten, Dachmembranen, Gewebebeschichtungen und Fußbodenbeläge.

Die Erfindung soll nachfolgend durch Beispiele erläutert werden. Die Beispiele sind ausgewählte Ausführungsformen und stellen keine Beschränkung dar.


Beispiel 1: Kernhydrierung von Diisononylphthalat (DINP)

Es wurden Versuche zur Kernhydrierung von DINP durchgeführt, bei der der Umsatz von DINP in Abhängigkeit der Zeit gemessen wurde. Hierzu wurden folgende DINP-Muster getestet.

**DINP-1** ist das Produkt VESTINOL® 9 der Firma Evonik Oxeno GmbH & Co. KG. Die CO-Zahl wurde anhand des in der Beschreibung genannten Verfahrens ermittelt und betrug 0,006 mg KOH/g.

**DINP-2** ist das Produkt eines koreanischen Unternehmens. Die CO-Zahl wurde anhand des in der Beschreibung genannten Verfahrens ermittelt und betrug 0,048 mg KOH/g

**DINP-3** ist das Produkt Kanatol-900 der Firma KLJ Group, Indien. Die CO-Zahl wurde anhand des in der Beschreibung genannten Verfahrens ermittelt und betrug 0,15 mg KOH/g.


Kernhydrierung der DINP-Proben

Es wurde eine Batchhydrierung von verschiedenen DINP-Proben (Diisononylphthalat) in einem Rohrreaktor mit einem Innendurchmesser von 40 mm und einer Länge von 250 mm im Kreislaufbetrieb durchgeführt. Der Rohrreaktor wird dabei im Gleichstrom mit Flüssigphase und Gasphase im Rieselbett durchströmt. Der bei der Hydrierung verwendete Katalysator war ein Schalenkatalysator bestehend aus 1 Gew.-% Ru, welches geträgert auf Titandioxid (Aerolyst 7711, Fa. Evonik Operations GmbH) vorliegt. In den Rohrreaktor wurde eine Mischung aus jeweils 25 g Hydrierkatalysator sowie 25 g Inertmaterial, bestehend aus $\gamma$-$Al_2O_3$ (Spheralite 538E. Fa. Axens), jeweils in Form von 1,5 mm Extrudaten, eingesetzt. Die bei der Hydrierung eingesetzte Menge an DINP betrug stets 1000 g. Die $H_2$-Regelung erfolgt über eine Abgasfahrweise, wobei ein konstanter Abgasstrom von 1 L/h (47,8 $m^3 m^{-2} h^{-1}$) eingestellt wurde. Alle Versuche wurden bei einem Anlagendruck von 90 bar sowie einer Rohrreaktortemperatur von 110 °C durchgeführt. Nach Passieren eines Wärmetauschers unterhalb des Reaktors erfolgte eine Gas-Flüssigtrennung mittels Abscheider. Die Gasphase wurde kontinuierlich ins Abgas entspannt. Über einen Zeitraum von 8 Stunden wurde die Flüssigphase über einen beheizten Vorwärmer stets zum Rohrreaktor zurückgeführt. Der Reaktionsfortschritt (Umsatz an DINP über die Zeit) im Reaktor wurde mittels inline-Raman-Analytik erfasst.

Die Ergebnisse sind der nachfolgenden Tabelle 1 zu entnehmen.

Tabelle 1: DINP-Umsatz (%) zu DINCH in Abhängigkeit von der Zeit

| Probe | CO-Zahl | Umsatz DINP 0h | Umsatz DINP 1h | Umsatz DINP 2h | Umsatz DINP 4h | Umsatz DINP 8h |
|-------|---------|----------------|----------------|----------------|----------------|----------------|
| DINP-1 | 0,006 | 0 | 32 | 57 | 87 | 99 |
| DINP-2 | 0,048 | 0 | 28 | 55 | 83 | 99 |
| DINP-3 | 0,15 | 0 | 22 | 40 | 65 | 90 |

Aus der Tabelle ist zu entnehmen, dass bei Einsatz von DINP mit einer CO-Zahl innerhalb des beanspruchten Bereichs (DINP-1 und DINP-2) eine deutlich schnellere Kernhydrierung erfolgt.

**Patentansprüche**

1. Verfahren zur Herstellung von 1,2-Cyclohexandicarbonsäuredialkylestern, bei denen die beiden Alkylgruppen jeweils 8 oder 9 Kohlenstoffatome aufweisen, wobei das Verfahren mindestens die folgenden Schritte umfasst

   a) Herstellen eines Dialkylphthalats durch Umsetzung von Phthalsäureanhydrid oder Dimethylphthalat mit einem C8- oder C9-Alkohol und Ausschleusen eines Teils einer während der Reaktion durch Anlegen eines Unterdrucks im Bereich von 500 bis 900 mbar absolut abdestillierten Leichtsiederphase, die zumindest nicht umgesetzten Alkohol und Reaktionsnebenprodukte umfasst,
   b) Kernhydrieren des in Schritt a) hergestellten Dialkylphthalats, bei dem die beiden Alkylgruppen jeweils 8 oder 9 Kohlenstoffatome aufweisen, in Anwesenheit eines heterogenen Hydrierkatalysators mit einem Wasserstoff-haltigen Gas zum entsprechenden 1,2-Cyclohexandicarbonsäuredialkylester umfasst, **dadurch gekennzeichnet, dass**

   das bei der Kernhydrierung in Schritt b) eingesetzte Dialkylphthalat eine CO-Zahl von weniger als 0,1 mg KOH/g, vorzugsweise weniger als 0,08 mg KOH/g, weiterhin bevorzugt weniger als 0,07 mg KOH/g aufweist und besonders bevorzugt weniger als 0,01 mg KOH/g aufweist.

2. Verfahren nach Anspruch 1, wobei die beiden Alkylgruppen des 1,2-Cyclohexandicarbonsäuredialkylesters jeweils 2-Ethylhexyl oder Isononyl sind.

3. Verfahren nach Anspruch 2, wobei der 1,2-Cyclohexandicarbonsäuredialkylester ein 1,2-Cyclohexandicarbonsäurediisononylester oder ein 1,2-Cyclohexandicarbonsäuredi-2-ethylhexylester ist.

4. Verfahren nach einem der Ansprüche 1 bis 5, wobei der bei der Kernhydrierung eingesetzte heterogene Hydrierkatalysator ein Übergangsmetall auf einem Trägermaterial umfasst.

5. Verfahren nach Anspruch 4, wobei das Übergangsmetall ein Metall der Gruppe 8 des Periodensystems der Elemente (Eisengruppe), vorzugsweise Ruthenium ist.

6. Verfahren nach Anspruch 4, wobei das Trägermaterial aus der Gruppe, bestehend aus Aktivkohle, Siliciumcarbid, Aluminiumoxid, Siliciumoxid, Alumosilikat, Titandioxid, Zirkoniumdioxid, Magnesiumoxid, Zinkoxid oder Mischungen davon, ausgewählt wird.

7. Verfahren nach Anspruch 6, wobei das Trägermaterial Titandioxid oder Aluminiumoxid ist.

8. Verfahren nach einem der Ansprüche 4 bis 7, wobei der Gehalt an Übergangsmetall im heterogenen Hydrierkatalysator im Bereich von 0,1 bis 10 Gew.-%, vorzugsweise insbesondere im Bereich von 0,3 bis 5 Gew.-%, besonders im Bereich von 0,5 und 3 Gew.-% liegt.

9.  Verfahren nach einem der vorherigen Ansprüche, wobei die Kernhydrierung in mindestens einer Hydriereinheit, vorzugsweise in mindestens zwei hintereinandergeschalteten Hydriereinheiten durchgeführt wird, wobei mindestens eine der mindestens zwei Hydriereinheiten in Schlaufenfahrweise betrieben wird.

10. Verfahren nach einem der vorherigen Ansprüche, wobei die Hydriertemperatur bei der Kernhydrierung im Bereich von 50 bis 250 °C liegt.

11. Verfahren nach einem der vorherigen Ansprüche, wobei die Kernhydrierung in einem Druckbereich von 3 bis 300 bar durchgeführt wird.

12. Verwendung der nach einem der Ansprüche 1 bis 11 hergestellten 1,2-Cyclohexandicarbonsäuredialkylester als Weichmacher oder als Teil einer Weichmacherzusammensetzung in Kunststoffen oder Kunststoffzusammensetzungen.

13. Verwendung der nach einem der Ansprüche 1 bis 11 hergestellten 1,2-Cyclohexandicarbonsäuredialkylester als Zusatz in Farben oder Lacken, in Klebstoffen oder Klebstoffkomponenten, in Dichtungsmassen oder als Lösemittel.

**Europäisches Patentamt**
**European Patent Office**
**Office européen des brevets**

# EUROPÄISCHER RECHERCHENBERICHT

Nummer der Anmeldung

EP 24 19 6456

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2006/167151 A1 (GRASS MICHAEL [DE] ET AL) 27. Juli 2006 (2006-07-27) * Beispiel 1 * | 1-11 | INV. C07C67/303 C07C69/80 C07C67/03 |
| X | EP 1 676 829 B1 (EVONIK OXENO GMBH [DE]) 5. Januar 2011 (2011-01-05) | 12,13 | C07C67/08 |
| Y | * Absatz [0066] - Absatz [0069]; Beispiel 1 * | 1-11 | |
| Y | DE 196 35 769 A1 (DEGUSSA [DE]) 5. März 1998 (1998-03-05) * das ganze Dokument * | 1-11 | |
| Y | CN 108 976 117 A (DALIAN INST CHEM & PHYSICS CAS) 11. Dezember 2018 (2018-12-11) * das ganze Dokument * | 1-11 | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| Den Haag | 2. Dezember 2024 | Fritz, Martin |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 24 19 6456

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2024

| Im Recherchenbericht angeführtes Patentdokument | Datum der Veröffentlichung | Mitglied(er) der Patentfamilie | Datum der Veröffentlichung |
|---|---|---|---|
| US 2006167151 A1 | 27-07-2006 | CN 1796353 A | 05-07-2006 |
| | | DE 102004063637 A1 | 13-07-2006 |
| | | EP 1676828 A2 | 05-07-2006 |
| | | ES 2330233 T3 | 07-12-2009 |
| | | JP 4944440 B2 | 30-05-2012 |
| | | JP 2006188521 A | 20-07-2006 |
| | | SG 123729 A1 | 26-07-2006 |
| | | TW I359808 B | 11-03-2012 |
| | | US 2006167151 A1 | 27-07-2006 |
| EP 1676829 B1 | 05-01-2011 | CN 1796352 A | 05-07-2006 |
| | | DE 102004063673 A1 | 13-07-2006 |
| | | EP 1676829 A2 | 05-07-2006 |
| | | JP 2006188520 A | 20-07-2006 |
| | | SG 123746 A1 | 26-07-2006 |
| | | SG 158123 A1 | 29-01-2010 |
| | | TW I360536 B | 21-03-2012 |
| | | US 2006161017 A1 | 20-07-2006 |
| DE 19635769 A1 | 05-03-1998 | CA 2214491 A1 | 04-03-1998 |
| | | DE 19635769 A1 | 05-03-1998 |
| | | EP 0827947 A1 | 11-03-1998 |
| | | JP H1095747 A | 14-04-1998 |
| | | US 5922900 A | 13-07-1999 |
| | | ZA 977915 B | 02-03-1998 |
| CN 108976117 A | 11-12-2018 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009095126 A1